# EUROPEAN PATENT SPECIFICATION

(11) **EP 1 456 356 B1**
(45) Date of publication and mention of the grant of the patent: **23.12.2009**
(21) Application number: 02785716.8
(22) Date of filing: 18.10.2002
(51) Int. Cl.: C12N 5/06

(54) **METHOD OF PRODUCING HUMAN BETA CELL LINES**
VERFAHREN ZUR HERSTELLUNG MENSCHLICHER BETA-ZELLLINIEN
PROCEDE DESTINE A PRODUIRE DES LIGNEES CELLULAIRES BETA HUMAINES

(30) Priority: 19.10.2001 US 981750
(43) Date of publication of application: 15.09.2004
(73) Proprietor: UNIVERSITE PARIS 7 - Denis DIDEROT, F-75005 Paris (FR); CENTRE NATIONAL DE LA RECHERCHE SCIENTIFIQUE (CNRS), 75016 Paris (FR)
(72) Inventor: CZERNICHOV, Paul, F-75020 Paris (FR); SCHARFMANN, Raphael, F-75005 Paris (FR); RAVASSARD, Philippe, F-75013 Paris (FR); MALLET, Jacques, F-75013 Paris (FR)
(74) Representative: Le Forestier, Eric
(86) International application number: PCT/IB2002/004599
(87) International publication number: WO 2003/033685

(56) References cited:
- CASTAING M ET AL: "Blood glucose normalization upon transplantation of human embryonic pancreas into beta-cell-deficient SCID mice." DIABETOLOGIA, vol. 44, no. 11, November 2001 (2001-11), pages 2066-2076, XP002235309 ISSN: 0012-186X
- OTONKOSKI TIMO ET AL: "Differentiation and maturation of porcine fetal islet cells in vitro and after transplantation." TRANSPLANTATION (BALTIMORE), vol. 68, no. 11, 15 December 1999 (1999-12-15), pages 1674-1683, XP009007988 ISSN: 0041-1337
- SANDLER S ET AL: "TISSUE CULTURE OF HUMAN FETAL PANCREAS. DEVELOPMENT AND FUNCTION OF B-CELLS IN VITRO AND TRANSPLANTATION OFEXPLANTS TO NUDE MICE" DIABETES, NEW YORK, NY, US, vol. 34, no. 11, 1 November 1985 (1985-11-01), pages 1113-1119, XP000651382 ISSN: 0012-1797
- BEATTIE GILLIAN M ET AL: "Functional beta-cell mass after transplantation of human fetal pancreatic cells: Differentiation of proliferation?" DIABETES, vol. 46, no. 2, 1997, pages 244-248, XP009008028 ISSN: 0012-1797
- MILO-LANDESMAN DALIT ET AL: "Correction of hyperglycemia in diabetic mice transplanted with reversibly immortalized pancreatic beta cells controlled by the tet-on regulatory system." CELL TRANSPLANTATION, vol. 10, no. 7, 2001, pages 645-650, XP009008067 ISSN: 0963-6897
- SALMON P ET AL: "REVERSIBLE IMMORTALIZATION OF HUMAN PRIMARY CELLS BY LENTIVECTOR-MEDIATED TRANSFER OF SPECIFIC GENES" MOLECULAR THERAPY, ACADEMIC PRESS, SAN DIEGO, CA,, US, vol. 2, no. 4, October 2000 (2000-10), pages 404-414, XP001028604 ISSN: 1525-0016
- EFRAT S: "Cell therapy approaches for the treatment of diabetes." CURRENT OPINION IN INVESTIGATIONAL DRUGS (LONDON, ENGLAND: 2000) ENGLAND MAY 2001, vol. 2, no. 5, May 2001 (2001-05), pages 639-642, XP001146486 ISSN: 1472-4472

## Description

### FIELD OF THE INVENTION

The invention relates to the field of biology and in particular to the field of cellular biology and cellular therapy.

### BACKGROUND OF THE INVENTION

Type I diabetes is due to the destruction by immune mechanisms of pancreatic beta cells, resulting in the lack of insulin production and hyperglycemia. Cell therapy using beta cells from donors could represent one way to cure diabetic patients. However, two main problems have to be solved before this goal can be reached. First, immunosuppressive protocols have to be designed to provide immunologic protection of the graft. Recent reports indicate that progress has been made in this field (Shapiro *et al*., 2000). The second point to be solved concerns the small number of mature beta cells from donors that are available for grafting (Weir and Bonner-Weir, 1997). There is a need to provide alternative sources of functional mature beta cells. That is the problem the present invention wishes to solve. During the last few years, it has been proposed that by understanding and recapitulating beta cell development that occurs during embryonic and fetal life, new beta cells could be produced that could be used for cell therapy of type I diabetes. Huge effort and progress have thus been made to define the molecular mechanisms that control prenatal pancreatic development in rodents and the role of specific transcription and growth factors has been defined (Edlund, 1998; St Onge *et al*., 1999; Wells and Melton, 1999; Scharfmann, 2000; Grapin-Botton and Melton, 2000; Kim and Hebrok, 2001). Different tissue sources potentially rich in precursor cells are also currently tested for their ability to differentiate into mature beta cells. Such cells derive either from fetal or neonatal porcine pancreas (Yoom *et al*., 1999; Otonkoski *et al*., 1999), or from fractions of human adult pancreas enriched in duct cells and that are thought to contain precursor cells (Bonner-Weir, 1997). So far, prenatal human pancreatic tissues (14-24 weeks) have been used unsuccessfully because all the tissues derived from fetuses were at late stages of development and were already quite mature when used in different assays (Tuch *et al*., 1984; Tuch *et al*., 1986; Sandla *et al*., 1985; Hayek *et al*., 1997; Goldrath *et al*., 1995). For instance, Tuch *et al*. (1984), Sandler *et al*. (1985), Goldrath *et al*. (1995) and Povlsen *et al*. (1974) used human pancreatic fragments of 14 to 24 weeks of development that have been engrafted into immunoincompetent mice with the goal of following endocrine tissue development. After a few weeks or months in recipient mice, all endocrine cell types were found when human tissues were removed (Tuch *et al*., 1984; Sandler *et al*., 1985). However, it is important to remember that between 14 and 24 weeks of development (the age of the tissue at the time of the transplantation), endocrine cells are already present and associated into islets of Langerhans (Bouwens et al., 1997; Stefan et al., 1983; Fukayama et al.; 1986; Miettinen et al.; 1992). Moreover, in these experiments using late human fetal tissues, when the quantity of insulin-expressing cells present in the graft was compared before and after transplantation, no clear increase in the beta cell mass was detected (Tuch et al.). It is consequently difficult to determine whether the human endocrine cells that were present after a few weeks or months in the mouse were newly formed endocrine cells or cells that existed before transplantation and did survive.
The present invention solves the above-mentioned problem of providing mature beta cells as it is further described below.

### DETAILED DESCRIPTION OF THE INVENTION

The present invention provides a method of producing functional human pancreatic cells , the method comprising the steps as defined in claim 1.
In a preferred embodiment, the NOD/*scid* animal is a mouse. Preferably the NOD/*scid* mice of the invention are of any age of development, preferably sufficiently old to perform a graft into the kidney capsule. Preferably, the NOD/*scid* mice are about of the 2 to 15 weeks of development, more preferably to 6 to 8 weeks of development. A NOD/*scid* animal is an animal lacking T- and B-lymphocytes and failing to generate either humoral or cell-mediated immunity.

An "effective amount" is an amount sufficient to effect beneficial or desired clinical results. An effective amount can be administered in one or more applications, although it is preferable that one administration will suffice. For purposes of this invention, an effective amount of pancreatic cells is an amount that is sufficient to produce differentiated pancreatic cells which are able to restore one or more of the functions of the pancreas. It is contemplated that a restoration can occur quickly by the introduction of relatively large numbers of pancreas cells, for example greater than 10⁹ cells. In addition, it is also contemplated that when fewer pancreatic cells are introduced, function will be restored when the pancreas cell or cells are allowed to proliferate *in vivo.* Thus, an "effective amount" of pancreatic cells can be obtained by allowing as few as one pancreas cell sufficient time to regenerate all or part of a pancreas. Preferably, an effective amount administered to the individual is greater than about 10¹ pancreas cells, preferably between about 10² and about 10¹⁵ pancreas cells and even more preferably, between about 10³ and about 10¹² pancreas cells. The effective amount of the animal pancreatic cells transplanted at step (c) of the method of the invention is more preferably between 10³ to 10¹² animal pancreatic cells.

Pancreatic tissue can be used in the methods of the present invention without further treatment or modification. Modifications are described below. For both modified and unmodified cells, it is preferred that single cell suspensions are obtained from the tissue. Cell suspensions can be obtained by methods known in the art, for example, by centrifugation and enzyme treatment. Pancreas tissue or cell suspensions can also be frozen and thawed before use. Preferably, the cells are fresh after isolation and processing.

Alternatively, the pancreatic cells of the present invention can be culture long-term *in vitro* to produce stable lines of pancreas-regenerating cells. As used herein, the term "*in vitro culture*" refers to the survival of cells outside the body. Preferably, the cultures of the present invention are "long-term" cultures in that they proliferate stably *in vitro* for extended periods of time. These stable populations of cells are capable of surviving and proliferating *in vitro* with an embryonic pancreatic phenotype (i.e. these cells will be "stem" cells). Methods of culturing various types of stem cells are known in the art. For example, WO 94/16059 describes long-term culture (greater than 7 months) of neuronal cells. Long-term- culture of other types of stem cells are also described in the art and can be applicable to the cells of the present invention. The pancreatic cells cultured *in vitro* can be genetically modified to express a gene of interest, such as a therapeutic gene.

Such Pancreatic beta cell preferably expresses insulin in response to glucose. *Moreover*, the *present invention provides a* functional pancreatic beta cell that expresses glucagon in response to glucose. Additionally, said functional pancreatic cell expresses and secretes digestive *enzymes*. Said cell is preferably a human cell.
The pancreatic cells of the present invention can be modified, for example, using particular cell culturing conditions or by genetic engineering techniques. This latter modification includes the introduction of a transgene into said cell, either integrated into the genome of said cell, or present as an extrachromosomal replicon. In one emnodiment, the method of producing functional animal pancreatic cell of the invention further comprises the step of genetically modifying the cells obtained at step (d) and/or further comprises the preliminary step of genetically modifying said animal pancreatic cells not older than 10 weeks of development excluding human embryonic pancreatic cells. By "genetically modifying" it is meant, introducing at least one transgene (i.e. an exogenous nucleic acids molecule) in said cell, said transgene being stably transmitted to the cell progeny. As used herein, by transgene it is means any nucleic acids molecule, more preferably any genomic DNA or RNA, recombinant DNA or RNA, or cDNA sequences, either single or double stranded.

Preferably, the transgene comprises at least a sequence of interest operatively linked to a promoter sequence to form an expression cassette that directs the expression of the gene of interest into pancreatic cells. Said transgene or expression cassette is either present in a linear form, but preferably said transgene or DNA cassette is part of a nucleic acid fragment or is cloned into a cloning and/or expression vector. "operatively linked" as used herein, includes reference to a functional linkage between a promoter and a second sequence (i.e. the sequence of interest), wherein the promoter sequence initiates and mediates the transcription of said DNA sequence corresponding to the second sequence. A "promoter" or a "promoter sequence" is a DNA regulatory region capable of binding RNA polymerase in a cell and initiating transcription downstream (3'direction) coding sequence. Within the promoter sequence will be found a transcription initiation site, as well as protein binding domains responsible for the binding of RNA polymerase. Eukaryotic promoters will often, but not always, contains TATA boxes and CAT boxes. Additional responsive elements can be found in the promoter sequence or in its vicinity, such as activating sequences ("enhancers"), inhibitory sequences ("silencers") and upstream sequences. Various promoters, including ubiquitous or tissue-specific promoters, and inducible and constitutive promoters may be used to drive the expression of the gene of interest. Preferably, the promoter is able to drive the expression of the gene of interest in at least the pancreatic cells, or in pancreatic cells at specific stage of development, such as in pancreatic cells not older than 10 days of development excluding human embryonic pancreatic cells, or in mature beta cells. More preferably, the promoter is the insulin gene promoter and more preferably is selected among the rat insulin gene promoter (Genebank accession N°GBJ0074B), the mouse insulin gene promoter (Genebank accession N°GBX04724), the human insulin gene promoter (Genebank accession N°GBAP001994). Alternatively the promoter is inducible by an antibiotic, such as tetracycline. In another embodiment, the promoter drives the expression of the gene of interest in a temperature dependent manner; in such case, the promoter is preferably the one of the gene coding for temperature sensitive mutant form of large T antigen of SV40.

According to the present invention, a " vector " is a replicon in which another polynucleotide segment is attached, so as to bring the replication and/or expression to the attached segment. A vector can have one or more restriction endonuclease recognition sites at which the DNA sequences can be cut in a determinable fashion without loss of an essential biological function of the vector. Vectors can further provide primer sites (e.g. for PCR), transcriptional and/or translational initiation and/or regulation sites, recombinational signals, replicons, selectable markers, etc. Examples of vectors include plasmids, phages, cosmids, phagemid, yeast artificial chromosome (YAC), bacterial artificial chromosome (BAC), human artificial chromosome (HAC), virus, virus based vector, such as adenoviral vector, lentiviral vector, and other DNA sequences which are able to replicate or to be replicated *in vitro* or in a host cell, or to convey a desired DNA segment to a desired location within a host cell. In a preferred embodiment the vector is a viral based vector, more preferably a lentivirus-based vector such as a HIV-1 based vector. Such a lentivirus-based vector is preferably used to transfect embryonic pancreatic cell of the invention.

The recombinant DNA technologies used for the construction of the expression vector according to the invention are those known and commonly used by persons skilled in the art. Standard techniques are used for cloning, isolation of DNA, amplification and purification; the enzymatic reactions involving DNA ligase, DNA polymerase, restriction endonucleases are carried out according to the manufacturer's recommendations. These techniques and others are generally carried out according to Sambrook *et al*. (1989).

The transgene and the vector according to the invention is introduced either into functional pancreatic beta cells or into pancreatic cells not older than 10 weeks of development, excluding human embryonic pancreatic cells by using known techniques, such as for example transfection by calcium phosphate precipitation, lipofection, electroporation, heat shock, microinjection into a pronucleus, "gene gun", use of artificial viral envelopes, viral infection. Such transgene or vector is either integrated by homologous recombination into the host genome or by random integration, or is present as an extra-chromosomal replicon.

As used herein, "sequence of interest" means any double stranded or single stranded DNA or RNA molecules. It can either be a genomic DNA fragment, such as gene(s), intron(s), exon(s), regulatory sequence(s)or combinations or fragments thereof, or a recombinant DNA molecule such as a cDNA gene for instance. When, the sequence of interest may encode a protein of interest, said sequence of interest may be a selection gene, a reporter gene, a therapeutic gene, an immortalizing gene, a transforming gene. Such sequence of interest may comprises any of the genetic elements necessary for the correct expression of this protein of interest in the host cell.

In one embodiment such "sequence of interest" encode for a protein of interest having for example a diagnostic or a research interest. Examples of such proteins having a research interest are the selection proteins. As used herein, "selection gene" means a gene that encodes a protein or a peptide (i.e. a selectable marker) that allows one to select for or against a molecule or a cell that contains it, often under particular conditions i.e. in presence of a selective agent. These selection proteins include but are not limited to products which provide resistance against otherwise toxic compounds (e.g., antibiotics). For example, the ampicillin or the neomycin resistance genes constitute genes encoding for selection marker of the invention. Those selection markers can be either positive or negative (see Capecchi et al., US 5 631 153). According to a preferred embodiment, said selection marker protein is a positive selection marker protein encoded by a gene selected in the group consisting of antibiotic resistance genes, hisD gene, *Hypoxanthine phosphoribosyl transferase* (HPRT) gene, guanine-phosphoribosyl-transferase (Gpt) gene. Said antibiotic resistance gene is selected in the group consisting of hygromycin resistance gene, neomycin resistance genes, tetracyclin resistance gene, ampicillin resistance gene, kanamycin resistance gene, phleomycin resistance gene, bleomycin resistance gene, geneticin resistance gene, carbenicillin resistance gene, chloramphenicol resistance gene, puromycin resistance gene, blasticidin-S-deaminase gene. In a preferred embodiment, said antibiotic resistance gene is a hygromycin resistance gene, preferably an *Escherichia coli* hygromycin-B phosphotransferase (hpt) gene. In this case, the selective agent is hygromycin. In another preferred embodiment, said antibiotic resistance gene is a neomycin resistance gene. In this case, the selective agent is G418. In another embodiment, the positive selection marker protein of the invention is His D; in that case, the selective agent is Histidinol. In another embodiment, the positive selection marker protein of the invention is Hypoxanthine phosphoribosyl transferase (HPRT) or Hypoxanthine guanosyl phosphoribosyl transferase (HGPRT); in that case, the selective agent is Hypoxanthine. In another embodiment, the positive selection marker protein of the invention is guanine-phosphoribosyl-transferase (Gpt); in that case, the selective agent is xanthine. It is also in the scope of the invention to use negative selection marker proteins. For example, the genes encoding for such proteins are the HSV-TK gene; in that case the selective agent is Acyclovir-Gancyclovir. For example, the genes encoding for such proteins are the Hypoxanthine phosphoribosyl transferase (HPRT) gene or the guanine-phosphoribosyl-transferase (Gpt) gene; in these cases, the selective agent is the 6-Thioguanine. For example, the gene encoding for such proteins is the cytosine deaminase; in that case the selective agent is the 5-fluoro-cytosine. Other examples of negative selection marker proteins are the viral and bacterial toxins such as the diphteric toxin A (DTA).

Examples of such proteins having a research interest are the reporter proteins. By "reporter gene" is meant any gene which encodes a product (i.e the reporter protein) whose expression is detectable. A reporter protein may have one of the following attributes, without restriction: fluorescence, enzymatic activity, or an ability to be specifically bound by a second molecule (*e.g.,* biotin or an antibody-recognizable epitope). Examples of reporter proteins are autofluorescent proteins such as the green fluorescence protein (GFP), the enhanced green fluorescence protein (EGFP), the red fluorescence protein (RFP), the blue fluorescence protein (BFP), the yellow fluorescence protein (YFP) and the fluorescent variants of these proteins. In a preferred embodiment, the gene of interest is a gene coding GFP. Examples of reporter proteins having enzymatic activity are β-galactosidase, β-glucoronidase, alcaline phosphatase, luciferase, alcohol deshydrogenase, chloramphenicol-acetyl transferase, peroxydase.

In another embodiment, the sequence of interest is a therapeutic gene. A "therapeutic gene" is a gene that corrects or compensates for an underlying protein deficit or, alternately, that is capable of down-regulating a particular gene, or counteracting the negative effects of its encoded product, in a given disease state or syndrome. For example, a therapeutic gene of the invention is a transcription factor or a hormone, a growth factor that will enhance pancreatic cells insulin secretion.

In another preferred embodiment, the sequence of interest is a transforming gene. By "transforming gene" it is meant gene that gives to the cells that contain it and in which it is expressed, the ability to have a transformed or cancerous phenotype. Transformed cells preferably presents the following characteristics: immortality (or unlimited growth), the lost of contact inhibition, the independance toward growth factors, tumorigenicity, the lost of the anchoring. Examples of transforming genes are cellular genes *ras, met, NGL* (ex-neu), bcl-X, telomerase gene, or viral gene, such as large T antigen of SV40, or its temperature sensitive mutant form. According to a preferred embodiment the gene of interest is a temperature sensitive mutant of the large T antigen of simian virus 40 (SV40).

In a preferred embodiment, the sequence of interest is an immortalizing gene. By "immortalizing gene" it is meant gene that gives to the cells that contain it and in which it is expressed, the ability to grow (almost) undefinitely. The cells transfected by an immortalizing gene are able to divide (almost) undefinitely, unlike normal cells which are programmed to divide a limited number of times (for example less tha 50 divisions for an adult fibroblast). Among immortalizing genes, one can recite *myc* and *myb* cellular genes, DNA virus gene, such as large T antigen, or a temperature sensitive mutant form of large T antigen E1A. Immortalizing genes generally encode for nuclear proteins that bind to DNA, either directly or indirectly via protein factors.

Beside the immortalization of the pancreatic cell of the invention by introducing a transgene encoding for an immortilizing gene into the cell, the present invention also encompasses the immortalization of the pancreatic cells by transfection with a compound selected in the group comprising a natural virus, a recombinant virus, or a fragment thereof, a virus based vector that comprises an immortilizing gene. Consequently the method of producing functional animal pancreatic cell of the invention further comprises the step of immortalizing said animal embryonic pancreatic cells not older than 10 weeks of development, excluding human embryonic pancreatic cells and/or further comprises the step of immortalizing the cells obtained at step (d). Such virus may be selected among lentivirus, simian virus SV40, Epstein-Bahr virus, Moloney leukemia virus. Preferably, the cell of the invention are transfected with a lentivirus based vector, more preferably a HIV-1 based vector.

The virus based vector of the invention more preferably, the HIV-1 based vector of the invention comprises an expression cassette as previously described that comprises a gene of interest operatively linked to a promoter, such as a rat insulin promoter or a human insulin promoter. In one embodiment, the HIV-1 based vector comprises a rapporter gene as a gene of interest, and more preferably the GFP gene operatively linked to the rat insulin gene promoter. In another embodiment the HIV-1 based vector of the invention comprises an expression cassette comprises an incogene as a gene of interest, operatively linked to the rat insulin promoter.

Multi-transgenic cells are also in the scope of the invention. Moreover, a pancreatic cell of the invention may be immortalized by the above mentionned coumpound and genetically modified to express another gene of interest.

The isolated functional pancreatic cells of the invention can be cultured *in vitro*. Suitable culture media are well known to those of skill in the art and may include growth factors or other compounds which enhance survival, proliferation or selectively promote the growth of certain sub-types of pancreatic cells such as alpha, beta, delta pancreatic cells.

Another embodiment of the present invention is the NOD/*scid* animal in which the embryonic pancreatic cells have been engrafted. Such NOD/*scid* animal comprises at least one functional pancreatic cell of the invention at any stage of development, which is derived from the engrafted embryonic pancreatic cell.

The present invention relates to the use of the NOD/*scid* animal of the invention to study and understand the development and the functioning of healthy or pathologic pancreas. Such animal constitutes an excellent model to understand and study pancreatic development, mainly human pancreatic development. Moreover, such animal would be useful to screen compounds able to modulate pancreas development or to modulate the regulation of glycemia, by modulating or by acting, for instance, on the insulin or glucagon expression, or on the expression of any targeted gene or protein involved in glycemia regulation. Such animal would be also useful to screen compounds able to modulate the expression of digestive enzymes. By "modulate", it is meant "enhance", "decrease", or "cancel".

Unless defined otherwise, all technical and scientific terms used herein have the same meaning as is commonly understood by one of the skill in the art to which this invention belongs.

The figures and examples presented below are provided as further guide to the practitioner of ordinary skill in the art and are not to be construed as limiting the invention in anyway.

### BRIEF DESCRIPTION OF THE FIGURES

**Figure 1****: Development of the human pancreas in NOD/*scid* mice.** (A) a pancreas at 8 weeks of development before transplantation. (B-E) the pancreases were grafted under the kidney capsule of NOD/*scid* mice and analyzed 7 days (B), 2 months (C), 6 months (D), and 9 months (E) later.
**Figure 2****: Evolution of the weight of the transplanted pancreas.**
   Grafts were removed at different time points after transplantation, finely dissected to remove the fat, and weighed. A total of 22 grafts were analyzed.
**Figure 3****: Histological analysis.** Human pancreas at 8 weeks of development before transplantation (A), and one month (B) and six months (C) after transplantation stained with an anti-pan cytokeratin antibody (revealed in green) or with an anti-vimentin antibody (revealed in red).
**Figure 4****: Development of the pancreatic endocrine tissue in NOD/*scid* mice.** Eight-week pancreas before grafting (A), and 7 days (B), one month (C), 2 months (D), 6 months (E) and 9 months (F) after transplantation.
   Insulin (revealed in green) and glucagon (revealed in red) immunostainings. The arrows in (A) represent 2 cells that stain positive for both insulin and amylase. In G and H are shown representative in hybridizations of a proinsulin probe on sections from 8-week human pancreas before grafting (G) and after 6-months engraftment (H).

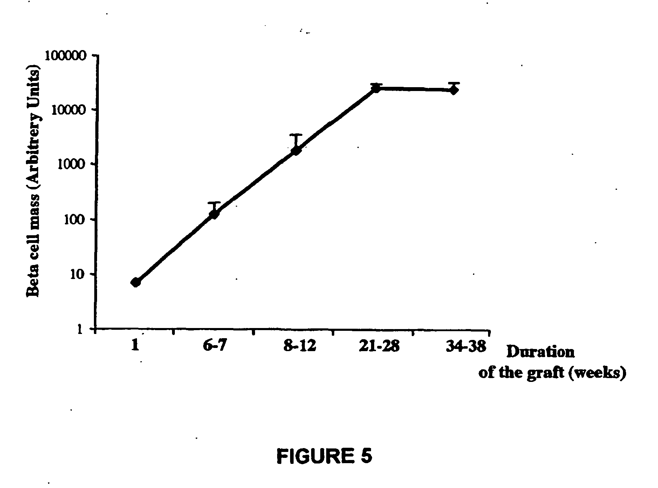

**Figure 5****: Evolution of the endocrine cell mass during the transplantation period.**
   The absolute mass of insulin-expressing cells is presented in arbitrary units. A total of 16 grafts were analyzed.

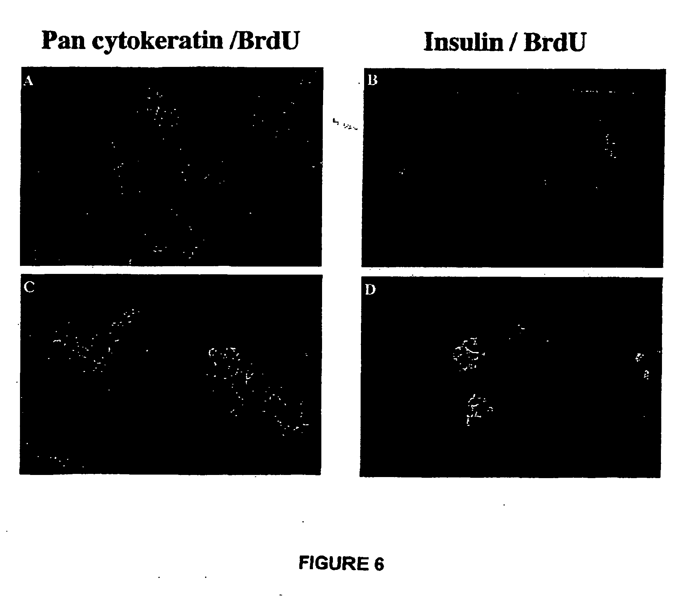

**Figure 6****: Cell proliferation analysis.**
   Double immunostaining for BrdU (red) and pancytokeratin (green) (A, C) or BrdU (red) and insulin (green) (B, D) on sections of human embryonic pancreas that developed in *scid* mice during 1 month (A and B) or during 3 months (C, D). Mice were sacrificed 2 hours after BrdU injection.

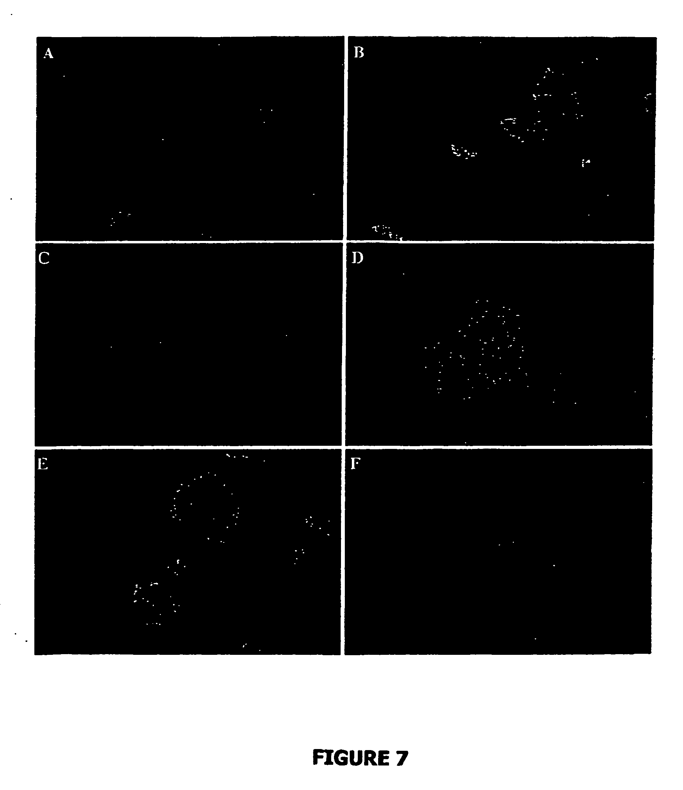

**Figure 7****: Human endocrine cells developed in mice resemble mature endocrine cells.**
   Sections of a human embryonic pancreas 6 months after transplantation. (A). Insulin (revealed in green) and Pax 6 (revealed in red); (B). Insulin (revealed in green) and Nkx6.1 (revealed in red); (C, D). Insulin (revealed in red) and PC1/3 (revealed in green); (E). Insulin (revealed in green) and Cytokeratin-19 (revealed in red); (F). Cytokeratin 19 alone (revealed in red).

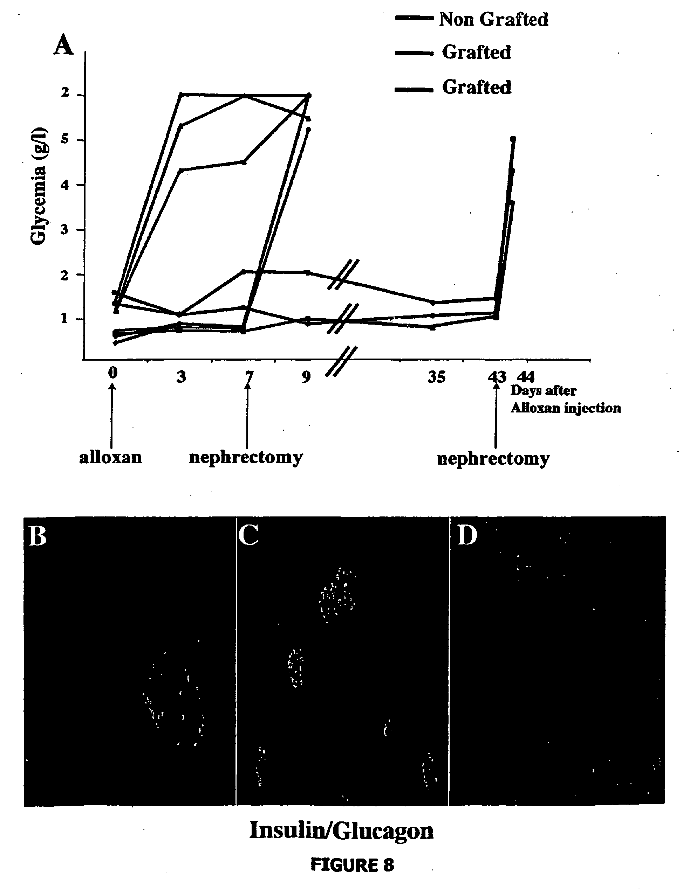

**Figure 8****: Functional development of the human pancreas graft.**
   (A) Three months after transplantation, *scid* mice (red lines) were injected with alloxan. Non-grafted mice (blue line) also received alloxan. While the glycemia of the non-grafted mice increased rapidly, that of the grafted mice remained stable. When grafts were removed by nephrectomy at day 7 or day 43, glycemia increased rapidly.
   (B) Mouse pancreas before alloxan treatment and at the end of the experiments (C) (day 43 after alloxan) stained for insulin (revealed in red) and glucagon (revealed in green), indicating that alloxan has destroyed the vast majority of host-insulin-expressing cells.(D). Section of the human graft at the end of the experiment (day 43 after alloxan) stained for insulin (revealed in red) and glucagon (revealed in green), indicating that alloxan had no effect on human beta cells that developed.

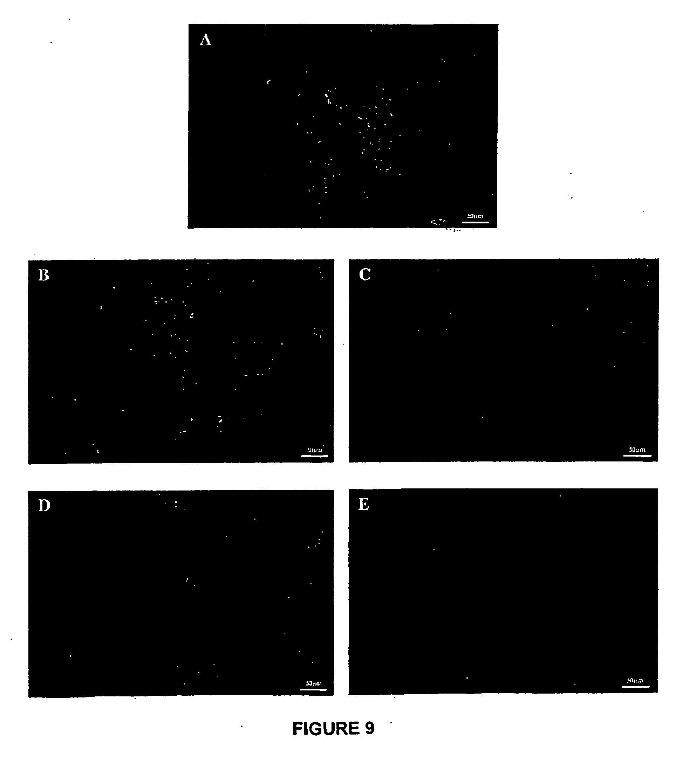

**Figure 9****: Development of rat embryonic pancreases engrafted in Scid mice and search for conditions to transduce insulin-expressing cells using recombinant lentiviruses.**
   E16 rat pancreases were dissected. In **A.,** they were immediately stained for glucagon (green) and insulin (red). In **B.** and **C.,** they were infected with lentiviruses expressing GFP under the control of the insulin promoter and grafted during 7 days. **B.** staining for insulin (red) and glucagon (green). **C.** No GFP-expressing cells could be detected under such conditions. In **D.** and **E.,** E16 pancreases were dissociated and next infected with lentiviruses expressing GFP under the control of the insulin promoter. **D.** staining for insulin (red) and glucagon (green). **E.** GFP-expressing cells could be detected under such conditions.

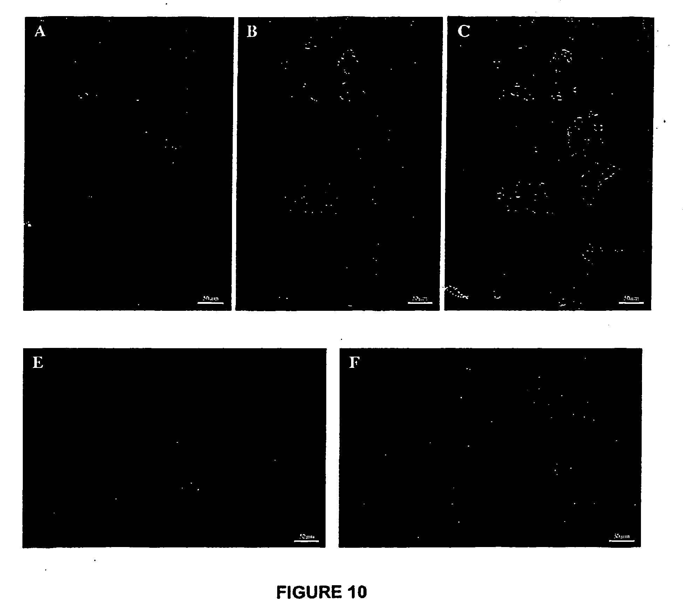

**Figure 10****: Expression of GFP in insulin-expressing cells.**
   E16 pancreases were dissociated and next infected with lentiviruses expressing GFP under the control of the insulin promoter and grafted during 7 days. **A.** GFP staining; **B.** insulin staining; **C.** Panels A and B were superimposed. **D.** Staining for glucagon (red) and GFP (green); **E.** Staining for Cytokeratin (red) and GFP (green).

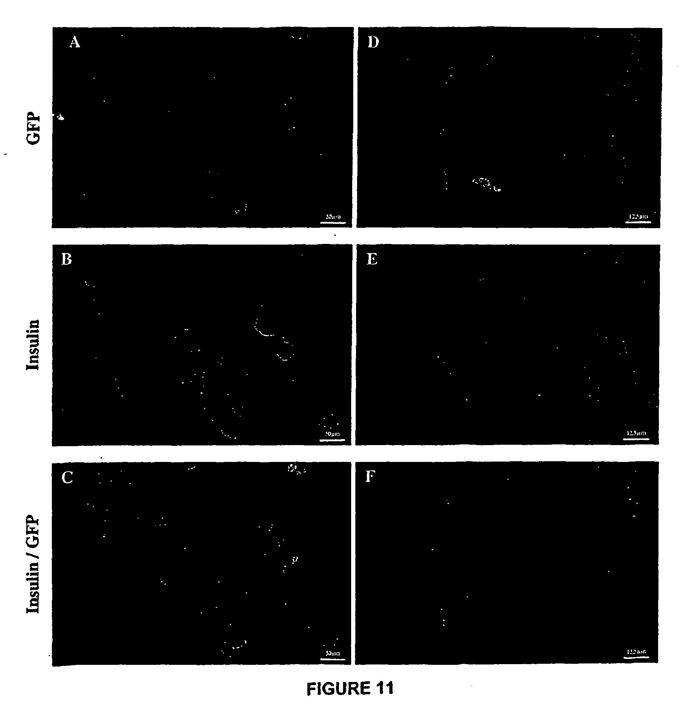

**Figure 11****: Long term expression of GFP in insulin-expressing cells.**
   E16 pancreases were dissociated and next infected with lentiviruses expressing GFP under the control of the insulin promoter and grafted during 1 Month. **A, D.** GFP staining; **B, E.** insulin staining; **C, F.** Panels A+B and D+E were superimposed.

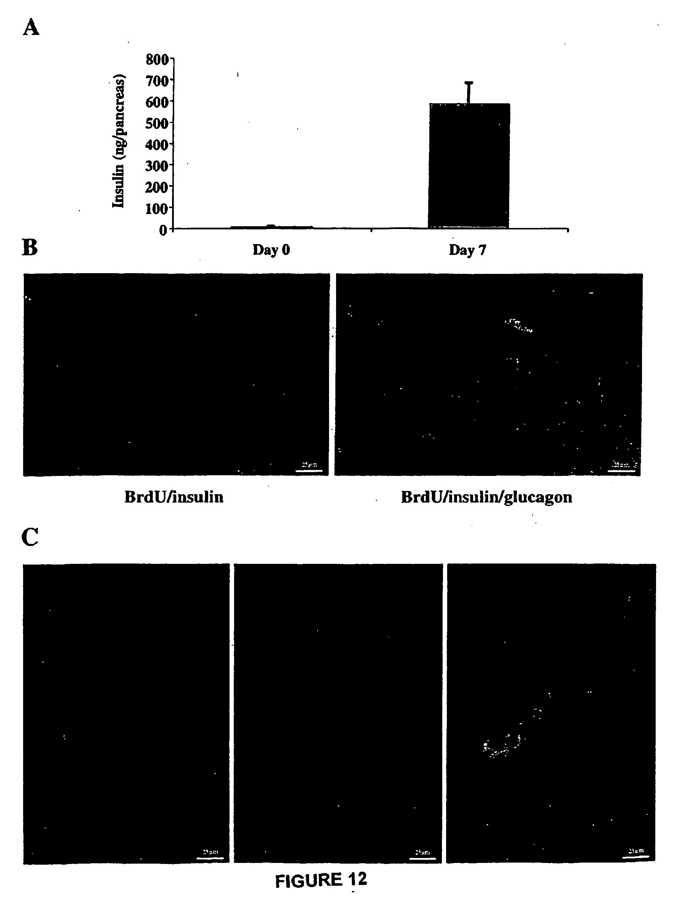

**Figure 12****: GFP⁺/INS⁺ cells develop from infected progenitor cells.**
   **A.** Insulin content in E16 pancreases before (day 0) or following a 7 day-graft (Day 7). **B.** Pregnant rats at E16 were injected with BrdU. On the left panel, the tissues were stained for BrdU (in red) and insulin (in green). On the right panel, the tissues were stained for BrdU (red), insulin (blue) and glucagon ((green). Note that the endocrine cells do not proliferate. **C.** E13 pancreases were dissociated and next infected with lentiviruses expressing GFP under the control of the insulin promoter and grafted during 7 days. **Left.** GFP staining; **Middle.** insulin staining; **Right.** Left and right panels were superimposed.

### EXAMPLES

### 1 - RESEARCH DESIGN AND METHODS

### 1.1 Human tissues

Human pancreases were dissected from embryonic tissue fragments obtained immediately after voluntary abortions performed between 6 and 9 weeks of development (WD), in compliance with the current French legislation and the guidelines of our institution. The warm ischemia time was less than 30 min. Gestational ages were determined from several developmental criteria: duration of amenorrhea; crown-rump length measured by ultrasound scan; hand and foot morphology. Pancreases were dissected and either fixed and embedded in paraffin or grafted to non-obese diabetic/severe combined immunodeficiency (NOD/*scid*) mice as described below.

### 1.2 Animals and transplantation into NOD/scid mice.

NOD/*scid* mice were bred in isolators supplied with sterile-filtered, temperature-controlled air. Cages, bedding and drinking water were autoclaved. Food was sterilized by X-ray irradiation. All manipulations were performed under a laminar flow hood. Embryonic pancreases (6-9 weeks of development (WD)) were implanted, using a dissecting microscope, under the left kidney capsule of 6- to 8- week-old NOD/*scid* mice that had been anaesthetized with Hypnomidate (Janssen-Cilag). At different time points after the graft (7 days - 9 months), mice were sacrificed and the grafts were removed, weighed, fixed in formalin 3.7 % and embedded in paraffin. For cell proliferation analysis, mice were injected with Bromo-deoxy Uridine (BrdU) (50mg/kg) 2 hours before sacrifice.

### 1. 3 Immunohistochemistry.

Four µm-thick sections were cut on gelatinized glass slides. For immunostaining, sections were deparaffinized in toluene, rehydrated, microwaved in citrate buffer 0.01 M, pH 6, and permeabilized for 20 min in Tris-Buffered Saline (TBS) containing 0.1 % Triton. Non-specific sites were blocked for 30 min in TBS containing 3% BSA and 0.1 % Tween 20 and sections were incubated overnight at 4°C with primary antibodies. The sections were then washed and incubated lh at room temperature with the appropriate secondary antibodies, labeled with 2 different fluorochromes. The primary antibodies were: mouse anti-human insulin (Sigma Aldrich, 1/1000); guinea pig anti-pig insulin (Dako; 1/2000); mouse anti-human glucagon (Sigma Aldrich, 1/2000); rabbit anti-human pan-cytokeratin (Dako, 1/500); mouse anti-human cytokeratin 19 (Dako; 1/50); mouse anti-pig vimentin (Dako; 1/30); rabbit anti-proconvertase 1/3 (gift from Dr Steiner, 1/200); rabbit anti-Pax6 (gift from Dr S. Saule); rabbit anti-rat Nkx6.1 (gift from Dr Serup); Mouse anti-BrdU (Amersham). Fluorescent secondary antibodies were: fluorescein-anti-guinea pig antibodies (Dako, 1:500); fluorescein-anti-rabbit antibodies (Immunotech, 1/200); fluorescein-anti-mouse antibodies (Immunotech, 1/200); Texas-red-antimouse antibodies (Jackson, 1/200); Texas-red-anti-rabbit antibodies (Jackson, 1/200).

### 1.4 Surface quantification and statistical analysis.

All images were numerized using a Hamamatsu C5810 cooled tri-CCD camera. Pictures were made at the same magnification, and analyzed with the IPLab software (version 3.2.4, Scananalytics Inc.). For each transplanted tissue, sections were taken at regular intervals throughout the graft and stained for insulin. Three to four sections and 3-5 views per section were analyzed. The evolution of the beta cell mass during the transplantation period was calculated as the product of the surface that stained positive for insulin by the corresponding graft weight.

### 1.5 In situ hybridization.

For *in situ* hybridization, sections were deparrafinized, rehydrated and permeabilized in PBS containing 1% Triton X-100. Prehybridization was dope at 70°C in hybridization buffer (50% formamide, 5 X SSC, 5X Denhardts' solution, 250∼µg/ml yeast RNA, 500µg/ml herring sperm DNA). RNA probes were labeled with DIG-UTP by *in vitro* transcription using the DIG-RNA labeling kit (Boehringer Mannheim). Hybridization was initiated by addition of fresh hybridization buffer containing Ipg/ml probe and continued overnight at 70°C. Thereafter, the slides were washed with decreasing concentrations of SSC. Revelation was processed by immunohistochemistry. Non-specific sites were blocked with 2% blocking reagent (Boehringer Mannheim) in Tris 25mM pH7.5, NaCl 140mM, KCl 2.7mM Tween 20 0.1 % for 30 min at room temperature. Slides were then incubated overnight at 4°C with alkaline phosphatase-conjugated polyclonal sheep anti-DIG antibody (diluted 1:1000, Boehringer Mannheim). The reaction product was visualized by an enzyme-catalyzed color reaction using nitro blue tetrazolium and 5-bromo-4chloro-3-indolyl-phosphate medium (Bohringer Mannheim). Sections were incubated until the colored reaction product developed at the sites of hybridization. The slides were washed in H₂0, mounted and visualized on a Leitz DMRD light microscope (Leica). The probe used here corresponded to human proinsulin.

### 1.6 Test of induction of diabetes.

To determine the capacity of the graft to regulate the glycemia of the mouse, grafted and nongrafted NOD/*scid* mice were injected intravenously (i.v.) with alloxan (Sigma-Aldrich, 90mg/kg body weight), that is known to destroy rodent, but not human, beta cells (Eizirik *et al*., 1994). Glucose levels were measured on blood collected from the tail vein every day during one week, using a portable glucose meter (GlucoMen, A. Menarini diagnostics, Firenze, Italy). To confirm the contribution of the graft to the normalization of blood glucose values in the host, grafts were removed by unilateral nephrectomy at different time points (7 days or 42 days) after the injection of alloxan and blood glucose levels were measured.

### 1.7 Animals and dissection.

Pregnant rats were purchased from the Janvier breeding center (CERJ, Le Genet, France). The morning of the discovery of the vaginal plug was designated as embryonic day 0.5 (E0.5). Pregnant rats were killed with CO₂, the embryos were harvested and the pancreases were dissected. For cell proliferation analysis, pregnant rats were injected with BrdU (100 mg/kg) 30 minutes before killing.

### 1.8 Construction and preparation of HIV-RIP-GFP vector: DNA constructs and vector production.

The promoterless pTRIP GFP has been described previously (Zennou *et al*., 2000). A 408 bp fragment of the Rat insulin promoter (RIP408) was produced from the pBS-RIP-beta globin plasmid (kindly provided by P. Herrera, (Sanvito et al., 1995)) by PCR using the expand system (Roche) with the following primers :
MluI RIP sens = 5' cgacgcgtGGACACAGCTATCAGTGGGA 3' (SEQ ID N°1)
BamHI RIP antisens =5' cgggatccTAGGGCTGGGGGTTACT 3' (SEQ ID N°2).
The resulting PCR product was subcloned into the pGEMT-easy vector (Promega). The MluI-BamHI fragment was then inserted into a MluI-BamHI linearized promoterless pTRIP GFP vector. To rule out point mutations induced by PCR the inserted RIP408 was entirely sequenced. Stocks of vectors were prepared by transitory co-transfection of 293T cells with the p8.7 encapsidation plasmid (ΔVprΔVifΔVpuΔNef) (Zufferey et al., 1997), pHCMV-G encoding the VSV envelope and the pTRIP RIP408-GFP vector (Yee et al., 1994), as described previously (Zennou et al., 2000). The supernatants were treated with DNAse I prior to ultracentrifugation then resuspended in PBS and frozen (-80°) until use.

### 1.9 Infection of embryonic pancreases:

Recombinant lentiviruses were used to infect either intact or dissociated pancreases. For dissociation, pancreases were incubated with 0.16 mg of Dispase I (Roche Bohringer) for 5 to 30 min depending of the size of the tissue and mechanically dissociated. For infection, the intact or dissociated tissue were incubated for one hour at 37°C with 200 µl of RPMI medium 1640 containing penicillin (100 units/ml), streptomycin (100 mg/ml), L-glutamin (2mM), and 10µl of viral particles (12 pg/ml of p24). To increase the rate of viral infection, DEAE-dextran was added at the concentration of 10 µg/ml.

### 1.10 Transplantation:

Scid mice were kept in isolators supplied with sterile-filtered, temperature-controlled air. Cages, bedding and drinking water were autoclaved. Food was sterilized by X-ray irradiation. All manipulations were performed under a laminar flow hood. Embryonic dissociated pancreases were implanted using a dissecting microscope under the left kidney capsule as previously described (Castaing et al., 2001), with the following modifications. The left kidney was exteriorized and a small transverse incision was made through the capsule on the ventral surface of the kidney, near the inferior pole. A pocket was created under the capsule, and a small silicon ring was partially pushed into the pocket under the capsule to provide a chamber for the transplanted tissue (Thomas et al., 1997). The dissociated cells were then introduce into the cylinder using a 50 µl Hamilton syringe with a blunt 22 gauge needle, and the ring fully introduced into the pocket, so that the capsule on the top and the kidney parenchyma on the bottom formed a sealed space contained the pancreatic cells. This method confines the cells to a single position so that the development could be easily studied. At different time points after transplantation, the mice were killed with CO₂, the kidney removed, and the graft were fixed in formalin 3,7% and embedded in paraffin.

### 1.11 Immunohistochemistry:

Four micrometer-thick were collected and analyzed by immunohistochemistry as described elsewhere (Cras-Meneur et al., 2001; Miralles et al., 1998). The antisera were used in the following dilutions: mouse anti-Human insulin (Sigma), 1/2,000), mouse anti-Human glucagon (Sigma), 1/2000; mouse anti-human pancytokeratin (Sigma), 1/100; mouse anti-BrdU (Amersham), 1/4); rabbit anti-human pancytokeratin (Dako), 1/500; rabbit anti-glucagon (DiaSorin), 1/2000; rabbit anti-porcine insulin (DiaSorin), 1/2000; and rabbit anti-GFP (Clontech), 1/200. The fluorescent secondary antibodies from Jackson Immunoresearch Laboratories were FITC anti-rabbit antibodies, 1/200; and Texas red anti-mouse antibodies, 1/200. For double-labeling immunofluorescence, antibodies raised in different species were applied to the sections and revealed by using antispecies antibodies labeled with two different fluorochromes. Single-labeled sections incubated with mismatched secondary antibodies showed no immunostaining, confirming the specificity of the secondary antisera. Photographs of the sections were taken using a fluorescence microscope (Leica, Leitz DMRB). They were digitized by using a Hamamatsu (Midlesex, NJ) C5810 colled 3CCD camera.

### 1.12 Insulin content:

For insulin content determination, embryonic pancreatic buds or grafted pancreases were homogenized. Insulin was extracted overnight in 10 ml of cold acidified ethyl alcohol (1,5% HCl, 75% EtOH) at -20°C. The extracts were stored at -20°C until assayed for insulin.
Immunoreactive insulin was measured with an Radioimmunoassay, using monoiodated ¹²⁵I-labeled porcine insulin (Sorin Biomedica, Sallugia, Italy) as a tracer, guinea-pig anti-insulin antibody kindly provided by Dr Van Schravendijk (Brussels, Belgium) and purified rat insulin (Novo Nordisk, Boulogne, France) as standard. Charcoal was used to separate free from bound hormone. The sensitivity of the assay was 0.25 ng/ml.

### 2 - RESULTS

### 2.1 Human embryonic engraftment in NOD/scid mice.

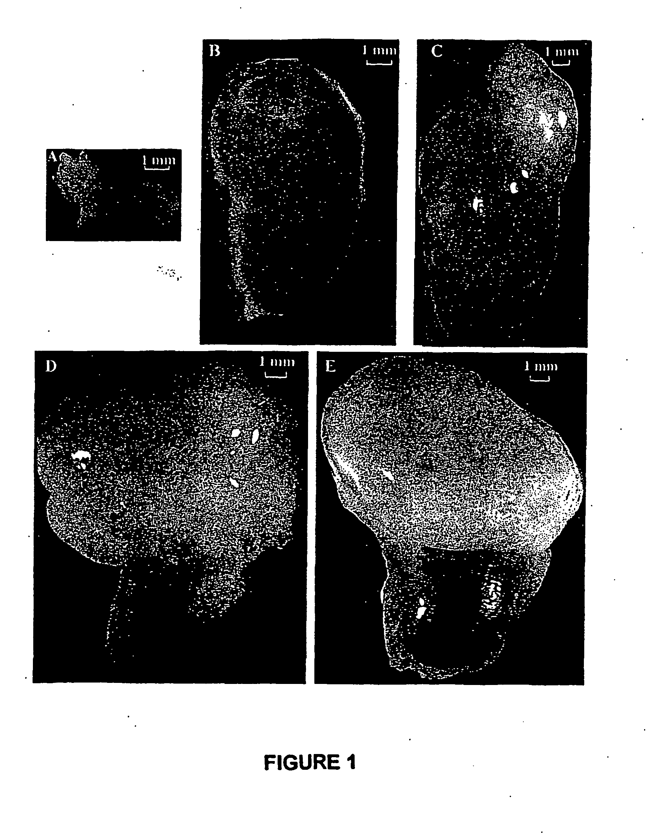

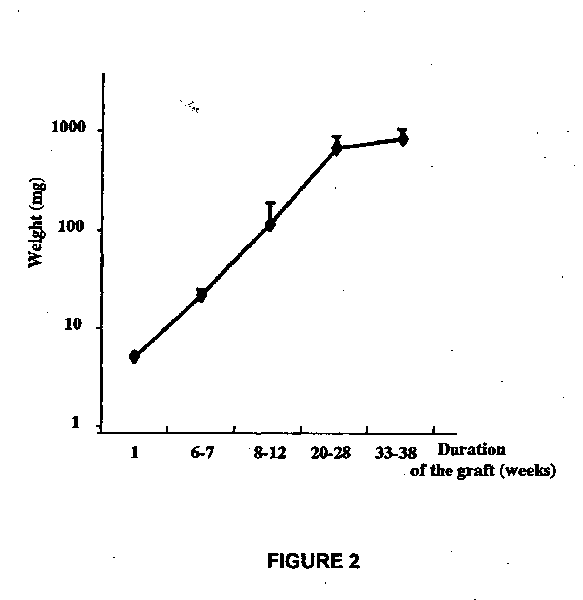

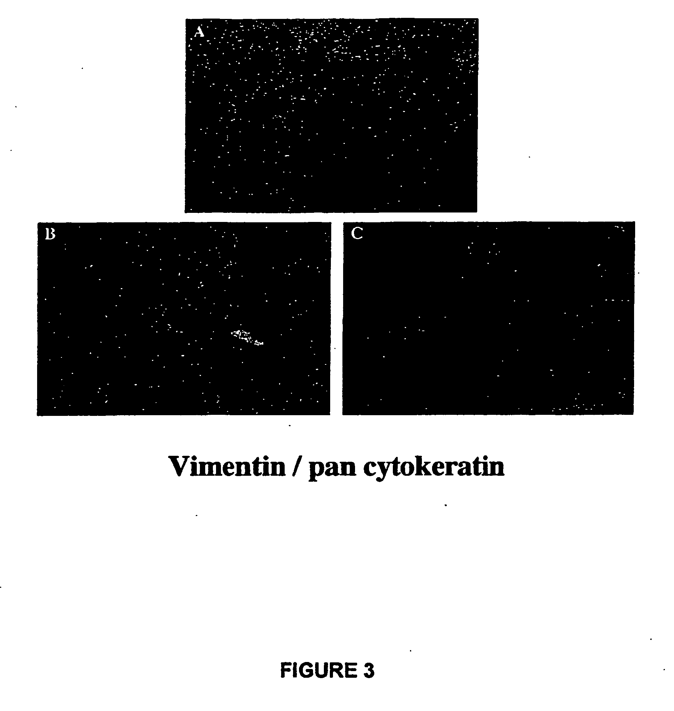

In the present study, 48 embryonic pancreatic tissues (6-9WD) were grafted to NOD/*scid* mice. Mice were sacrificed at different time points after transplantation (7 days-9 months), and the grafts were dissected. Thirty-nine grafts were recovered. Among the 9 non-recovered grafts, one grafted mouse died, but the graft was present. In 8 cases, mice died for unknown reasons and graft growth was not analyzed. The evolution of grafted tissues in terms of volume and mass was next followed. As shown in Fig.1, the human embryonic tissue developed massively when grafted under the kidney capsule of the *scid* mice. While at 7-9 weeks of gestation, before grafting, the volume of embryonic pancreas was not more than 4 mm³ (Fig. 1A), it increased with time in the mouse host and reached a volume of a few cm³ 6 months later (Fig. 1B-E). The evolution of the grafted tissue in term of mass was also followed. While, after 1 week in the mouse, graft weight was less than 10 mg, in the same range of the ungrafted tissue, it next increased rapidly with time to reach 100 mg after 8-12 weeks and 1000 mg after 33-38 weeks (Fig. 2). Immunohistochemistry using anti-cytokeratin and anti-vimentin antibodies was performed to follow the evolution during the grafting period of the epithelial and mesenchymal cells present in the human embryonic pancreas before transplantation. As shown in Fig. 3A, before grafting, an 8WD pancreas is composed of epithelial cells forming ducts and mesenchymal cells. One month and 6 months after transplantation, the tissue was also composed of epithelial cells that stained positive for cytokeratin and mesenchymal cells positive for vimentin, indicating that both cell types did develop during the graft (Fig. 3B and C).

### 2.2 Development of the endocrine tissue.

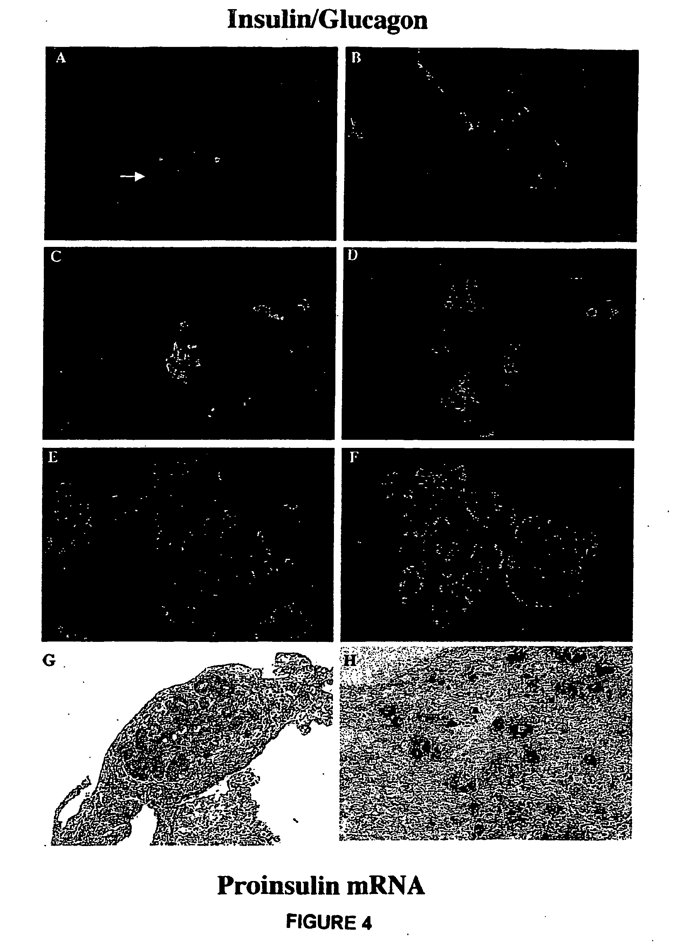

Before transplantation, only a few endocrine cells were detected by immunohistochemistry that stained positive either for glucagon, or for both insulin and glucagon. Such cells were dispersed in the pancreatic tissue and were not associated into islets of Langerhans (Fig. 4A). Once transplanted, the endocrine tissue started to develop and the number of endocrine cells increased with time (Fig. 4B-F). In panels G and H are shown representative hybridizations for proinsulin before and after transplantation for 6 months of an 8-week old pancreas. Huge increase in the number of cells that express proinsulin mRNA can be clearly visualized. Evolution of the insulin-positive cell mass was quantified after immunohistochemistry. As shown in Fig. 5, the absolute surface occupied by insulin-expressing cells was multiplied by 300 after 8-12 weeks in the mouse and by 5,000 after 21-28 weeks.

### 2.3 Human undifferentiated epithelial cells, but not endocrine cells, proliferated during the engraftment period.

To define whether the increase in the absolute number of endocrine cells was due to the differentiation of precursor cells or to the proliferation of the few endocrine cells that were present before grafting, engrafted mice were injected with BrdU 2 hours before sacrifice and immunohistological analysis was performed. As shown in Fig. 6, after both 1 and 3 months of development in the mouse, while cells that stained positive for both cytokeratin and BrdU were frequently detected, cells positive for both insulin and BrdU were very rarely found. These results strongly suggest that increase in the endocrine cell mass was due to the differentiation of precursor cells, rather than to the proliferation of rare preexisting endocrine cells.

### 2.4 Human endocrine cells developed in mice resemble mature endocrine cells.

To define whether human endocrine cells that developed in NOD/*scid* mice express markers known to be present in human beta cells developed *in vivo*, a series of antibodies were tested by immunohistochemistry. As shown in Fig. 7, beta cells developed in NOD/*scid* mice express specific transcription factors such as Nkx6.1 and Pax6 (Panels A, B), as well as PC 1/3, an enzyme necessary for the processing of proinsulin into insulin (panels C, D). Moreover, endocrine cells in the grafts are frequently associated in islets of Langerhans, with a core of insulin-expressing cells surrounded by glucagon-expressing cells (Fig. 4, panels E, F). Finally, while as described previously (Bouwens *et al*., 1997), the first endocrine cells found in human embryonic pancreas stain positive for cytokeratin, the human insulin-expressing cells that developed in NOD/*scid* mice did not express cytokeratin 19 (Fig. 7, panels E, F).

### 2.5 Functional development of human pancreas grafts.

To define whether the human beta cells that developed in the grafts were functional, 15 NOD/*scid* mice were engrafted with human embryonic pancreas. Three months later, grafted or non-grafted NOD/*scid* mice were injected with alloxan, a drug known to be toxic for murine, but not for human beta cells (Eizirik *et al*., 1994). Before alloxan injection, blood glucose levels were not statistically different in the transplanted and non-transplanted mice. After alloxan treatment, the glycemia of all non-grafted mice increased up to 6g/l. Conversely, the glycemia remained stable in 12 out of 15 engrafted mice. To demonstrate that glycemia regulation in engrafted mice injected with alloxan is indeed due to the development of the graft, unilateral nephrectomies were performed to remove the grafts in 6 mice and blood glucose levels were monitored. As shown in Fig. 8A, after removal of the graft by unilateral nephrectomy, either 7 or 43 days after alloxan injection the mice became hyperglycemic. Murine pancreases and grafts were also analyzed for the presence of insulin- and glucagon-expressing cells before alloxan injection, or at the end of the experiments when the animals were sacrificed. As shown in Fig. 8, very few insulin-expressing cells were detected in the murine pancreas of grafted NOD/*scid* mice that had been injected with alloxan, compared to NOD/*scid* mice that were not treated with alloxan. On the other hand, a huge amount of insulin-producing cells was present in the human graft after alloxan treatment.

The inventors demonstrate that human early embryonic pancreas can develop when engrafted under the kidney capsule of NOD/*scid* mice. The size and weight of the grafts increased considerably and endocrine cells differentiated which were organized into islets of Langerhans and showed numerous criteria of maturity. Finally, the human endocrine pancreatic tissues that developed could reverse diabetes in mice, indicating its functionality.

In the present study, the inventors used NOD/*scid* mice as recipients for transplantation. NOD/*scid* mice were generated by crossing the *scid* mutation from C.B-17-*scid*/*scid mice* onto the NOD background. These animals are lacking T- and B-lymphocytes, (Shultz *et al*., 1995), and fail to generate either humoral or cell-mediated immunity. Because of the absence of xenograft rejection in *scid* mice, they were previously used as recipients for human or fetal hematolymphoid tissues and cells (Roncarolo *et al*., 1995). Non hematopoietic human tissues such as ovarian cortex (Weissman *et al*., 1999), thyroid (Martin *et al*., 1993), skin (Levy *et al*., 1998) and airway (Delplanque *et al*., 2000) were also successfully transplanted in this model. However, the capacity of these tissues to develop functional properties and to replace a physiological function has been demonstrated only rarely. One example of physiological function replacement is the demonstration that adrenocortical tissue can form by transplantation of bovine adrenocortical cells and replace the essential functions of the mouse adrenal gland (Thomas *et al*., 1997). However, in this work, the transplanted tissue was not of human but of bovine origin. Moreover, the tissue had been expanded from a primary culture of bovine adrenocortical cells. Donor cells were thus already fully differentiated at the time of grafting.

In the present invention, the inventors demonstrate that immature human embryonic pancreas can develop and acquire functional properties in *scid* mice.

By grafted immature rudiments that contained undifferentiated epithelial cells and mesenchymal tissue and almost no insulin-expressing cells, the inventors demonstrate that the absolute mass of insulin-expressing cells was multiplied by nearly 5,000 after 6 months in the mouse. The fact that very few endocrine cells were present before transplantation, while a massive amount of endocrine cells was detected a few weeks later, clearly indicates that neoformation of endocrine cells occurred in the present model.

Theoretically, the observed increase in the human beta cell mass could be due either to the proliferation of rare preexisting insulin-expressing cells, or to the differentiation of precursor cells. It is thought that during prenatal life, increase in the beta cell mass in mainly due to the differentiation of precursor cells rather than to the proliferation of preexisting beta cells. This is quite clear in rodents where a large number of experiments have been performed that indicate that the increase in the endocrine cell mass observed during fetal life cannot be explained by the proliferation of preexisting endocrine cells (Swenne, 1992)). While less information is available, this seems to be also the case in humans, where, during embryonic/fetal life, insulin-expressing cells stain rarely positive for Ki67, and hence are rarely or not cycling (Potak *et al*., 2000; Bouwens *et al*., 1997). The inventors' data indicate that when chimeric mice are injected with BrdU, a large number of cytokeratin-positive cells present in the graft stain positive for BrdU, while no or very rare insulin-positive cells do. Thus, it can be postulated that in the grafts newly formed beta cells derived from precursor cells present in the duct epithelium that did proliferate and differentiate during engraftment rather than from proliferation of the few endocrine cells present in the rudiment, a mechanism that does recapitulate normal development.

Different arguments indicate that the human beta cells that did develop *in vivo* in NOD/*scid* mice are mature. First, these insulin - expressing cells did not coexpress glucagon and are thus different from the first insulin - expressing cells detected in the human pancreas at early stages of development (Polak *et al*., 2000 ; Larsson and Hougaard, 1994 ; De Krijger *et al*., 1992). Next, it has been shown that the insulin - expressing cells present in the pancreas before 16 weeks of development express cytokeratin 19, while the insulin-expressing cells found later during development stain negative for this marker (Bouwens *et al*., 1997). The inventors' data indicate that the human beta cells that develop in NOD/*scid* mice stain negative for cytokeratin 19 and do thus resemble adult mature beta cells. Next, human beta cells that develop in NOD/*scid* mice express the prohormone convertase PC 1/PC3, an enzyme that is necessary for the processing of proinsulin into insulin (Kaufman *et al*., 1997; Furuta *et al*., 1998). Finally, the inventors' data indicate that the human endocrine cell mass that developed in NOD/*scid* mice is able to perfectly regulate the glycemia of NOD/*scid* mice deficient in endogenous beta cells, and hence is functional. These human endocrine cells remain functional and can regulate the glycemia of the mice for at least 43 days, the longest period tested before removing the graft.

The inventors demonstrate here that newly differentiated human beta cells that are able to regulate the glycemia of the host deprivated of its own beta cells can be produced from human early embryonic pancreas. Human embryonic pancreas does thus represent an alternative source of tissue useful to generate functional human beta cells for transplantation. Moreover, the model of mice grafted with human embryonic pancreas can now be used to progress in the study of the development of the human pancreas, a type of study that were difficult to perform due to the lack of human embryonic pancreases and of proper experimental systems.

### 2.6 Development of rat embryonic pancreases engrafted in Scid mice and search for conditions to transduce insulin-expressing cells using recombinant lentiviruses

The inventor first studied the capacity of pancreatic tissue to grow and develop when engrafted to *scid* mice. E16,5 pancreases were dissected and next transplanted under the kidney capsule of *scid* mice. Seven days after grafting, the mice were killed, and the grafts were removed. As previously shown (Rall et al., 1973), at E16, before grafting, the vast majority of the endocrine cells in the pancreas are glucagon-expressing cells, very rare insulin-expressing cells being present at this stage. Such insulin-expressing cells were found dispersed (Fig. 9A). On the other hand, when E16 pancreases were grafted for 7 days, insulin-expressing cells developed and were now found associated forming islets of Langerhans (Fig. 9B). The inventor next used an HIV-1 based vector, containing the green fluorescent protein (GFP) gene as reporter. GFP was driven by the rat insulin promoter (RIP), to restrict the expression of GFP to beta cells. E16 pancreases were infected and grafted during 7 days. As show in Fig. 9C, no GFP-expressing cells were detected under such conditions. The inventor next repeated the experiments but dissociated the tissue before infection and graft. As shown in Fig. 9D, under such conditions, the endocrine tissue developed properly. Moreover, a large number of cells expressed GFP when the tissue was dissociated with Dispase before infection (Fig. 9E). GFP was never detected in non infected tissues (data not shown).

### 2.7 Long term expression of GFP in insulin-expressing cells.

The next step was to define the cell types expressing GFP. Double immunohistochemistry was performed using anti-insulin, -glucagon and -cytokeratin, to determine whether the GFP-expressing cells were beta cells, alpha cells or duct cells respectively. As shown in Fig. 10, GFP was clearly detected in insulin-expressing cells. On the other hand, GFP was never detected in glucagon-expressing cells or cytokeratin-expressing cells. Finally as shown in Fig. 11, sustained expression of GFP during more than one month (the longest time point analyzed) could be achieved when GFP was driven by the rat insulin promoter.

### 2.8 Beta cells expressing GFP derive from infected progenitors.

Two sets of arguments indicate that beta cells expressing GFP derive from progenitors that were first infected with RIP-GFP and next differentiated into beta cells and not by infection of the few beta cells present at E16 that would next proliferate. First, in the model of development of E16 pancreas grafted under the kidney capsule of Scid mice, beta cell differentiation occurs. Indeed, as shown in Fig. 12A, during a 1 week grafting period, insulin content per pancreas measured by radio immunoassay was multiplied by more than 250 fold. As shown in Fig 12B, while at this stage, as expected, proliferation of pancreatic cells is high, insulin-expressing cells do not proliferate. To further demonstrate that insulin/GFP cells derive from infected progenitor cells and not from preexisting mature beta cells, infection was repeated using dissociated pancreases at E13. As previously shown, at this stage, very few insulin-expressing cells are present that are fully immature (Jackerott et al., 1996; Miralles et al., 1998; Pictet and Rutter, 1972). It is now established that such immature insulin-expressing cells will not give rise later to mature beta cells (Jensen et al., 2000). As shown in Fig. 12C, when dissociated E13 pancreases were infected with RIP-GFP and transplanted under the kidney capsule of Scid mice, beta cells expressing GFP could be detected one week later, further indicating that Insulin/GFP double positive cells derived from endocrine progenitors that were infected at E13.

### REFERENCES

Becker et al. (1994) J Biol Chem 269: 21234-21238
Bonner-Weir et al. (2000) Proc Natl Acad Sci USA 97: 7999-8004
Buchet et al. (2002) Mol Cell Neurosci 19: 389-401
Bouwens et al. (1997) Diabetologia 40: 398-404
Case et al. (1999) Proc Natl Acad Sci USA 96: 2988-2993
Castaing et al. (2001) Diabetologia 44: 2066-2076
Cras-Meneur et al. (2001) Diabetes 50: 1571-1579
Csete et al. (1995) Transplantation 59: 263-268
De Krijger et al. (1992) Dev Biol 153: 368-375
De la Tour et al. (2001) Mol Endocrinol 15: 476-483
Delplanque et al. (2000) J Cell Sci 113: 767-778
Edlund (1998) Diabetes 47 : 1817-1823
Eizirik et al. (1994) Proc Natl Acad Sci USA 91 : 9253-9256
Englund et al. (2000) Neuroreport 11: 3973-3977
Evans et al. (1999) Hum Gene Ther 10: 1479-1489
Flax et al. (1998) Biotechnol 16: 1033-1039
Flotte et al. (2001) Diabetes 50: 515-520
Fukayama et al. (1986) Differentiation 31 : 127-133
Gallichan et al. (1998) Hum Gene Ther 9: 2717-2726
Goldrath et al. (1995) Transplantation 59: 1497-1500
Grapin-Botton, Melton (2000) Trends Genet 16 : 124-130
Halban et al. (2001) Diabetes 50: 2181-2191
Hayek, Beattie (1997) J Clin Endocrinol Metab 82 : 2471-2475
Jackerott et al. (1996) J Histochem Cytochem 44:809-817 Jensen et al. (2000) Diabetes 49: 163-176
Ju et al. (1998) Diabetologia 41: 736-739
Kapturczak et al. (2002) Mol Ther 5: 154-160
Kaufmann et al. (1997) Diabetes 46 : 978-982
Kim, Hebrok (2001) Genes Dev 15: 111-127
Larsson, Hougaard (1994) Endocrine 2: 759-765
Leibowitz et al. (1999) Diabetes 48: 745-753
Levine et al. (1995) Transplantation proceedings 27: 3410
Levy et al. (1998) Gene Ther 5: 913-922
Martin et al. (1993) J Clin Endocrinol Metab 77: 305-310
May et al. (2000) Nature 406: 82-86
Miettinen, Heikinheimo (1992) Development 114: 833-840
Miralles et al. (1998) Development 125: 1017-1024
Miyoshi et al. (1999) Science 283: 682-686
Otonkoski (1999) Transplantation 68 : 1674-1683
Pawliuk et al. (2001) Science 294 : 2368-2371
Pictet et al. (1972) Development of the embryonic pancreas
Polak et al. (2000) Diabetes 49 : 225-232
Povlsen et al. (1974) Nature 248 : 247-249
Roncarolo et al. (1995) Georgetown TX : Landes
Rall et al. (1973) Proc Natl Acad Sci USA 70 : 3478-3482
Salmon et al. (2000) Mol Ther 2 : 404-414
Salton et al. (1991) Mol Cell Biol 11 : 2335-2349
Sandler et al. (1985) Diabetes 34 : 1113-1119
Sanvito et al. (1995) Biochem Biophys Res Commun 217 : 1279-1286
Shapiro et al. (2000) N Engl J Med 343 : 230-238
St-Onge et al. (1999) Curr Opin Genet Dev 9 : 295-300
Shapiro et al. (2000) N Engl J Med 343 : 230-238
Sharfmann (2000) Diabetologia 43 : 1083-1092
Shultz et al. (1995) J Immunol 154: 180-191
Stefan et al. (1983) Diabetes 32 : 293-301
Swenne (1992) Diabetologia 35 : 193-201
Thomas et al. (1997) Nat Med 3: 978-983
Tuch et al. (1984) Diabetes 33 : 1180-1187
Tuch et al. (1986) Diabetes 35 : 464-469
Uchida et al. (1998) Proc Natl Acad USA 95: 11939-11944
Verma et al. (1997) Nature 389: 239-242
Weir, Bonner (1997) Diabetes 46 : 1247-1256
Weissman et al. (1999) Biol Reprod 60: 1462-1467
Wells, Melton (1999) Annu Rev Cell Dev Biol 15: 393-410
Yee et al. (1994) Proc Natl Acad USA 91: 9564-9568
Yoon et al. (1999) Cell Transplant 8: 673-689
Zennou et al. (2000) Cell 101: 173-185
Zufferey et al. (1997) Nat Biotechnol 15: 871-875

## Claims

1. A method of producing functional human pancreatic cell wherein said method comprises the steps of:
(a) immortalizing human pancreatic tissue or single cell suspensions thereof not older than 10 weeks of development, excluding human embryonic pancreatic cells,
(b) introducing an effective amount of human pancreatic tissue or single cell suspensions thereof obtained in step (a) into the kidney capsule of non-obese diabetic/severe combined immunodeficiency (NOD/scid) non-human animal,
(c) allowing the human pancreatic cells to develop, to differentiate and regenerate at least a pancreatic function; and
(d) collecting human pancreatic cells obtained at step (b) at different periods of time, and
(e) optionally *in vitro* culturing the cells obtained at step (c).

2. Use of pancreatic cells obtained by the method of claim 1 for studying pancreas development.

3. Use of pancreatic cells obtained by the method of clam 1 for studying the physiopathological development of diabetics.

## Patentansprüche

1. Verfahren zur Herstellung einer funktionellen humanen Pankreaszelle, wobei das Verfahren die folgenden Schritte umfasst:
(a) Immortalisieren von humanem Pankreasgewebe oder Einzelzellsuspensionen davon, nicht älter als 10 Wochen ab der Entwicklung, unter Ausschluss humaner embryonaler Pankreaszellen,
(b) Einführen einer wirksamen Menge von humanem Pankreasgewebe oder Einzelzellsuspensionen davon, erhalten in Schritt (a), in die Nierenkapsel eines nicht-fettleibigen, diabetischen, nicht-humanen Lebewesens mit schwerem kombiniertem Immundefekt (NOD/scid),
(c) Entwickelnlassen der humanen Pankreaszellen, um wenigstens eine Pankreasfunktion zu differenzieren und zu regenerieren; und
(d) Sammeln von in Schritt (b) erhaltenen humanen Pankreaszellen in verschiedenen Zeiträumen, und
(e) optionales in-vitro-Kultivieren der in Schritt (c) erhaltenen Zellen.

2. Verwendung von Pankreaszellen, die durch das Verfahren nach Anspruch 1 erhalten werden, zur Untersuchung der Pankreasentwicklung.

3. Verwendung von Pankreaszellen, die durch das Verfahren nach Anspruch 1 erhalten werden, zur Untersuchung der physiopathologischen Entwicklung von Diabetes.

## Revendications

1. Procédé de production d'une cellule pancréatique humaine fonctionnelle, où ledit procédé comprend les étapes consistant à :
(a) immortaliser du tissu pancréatique humain ou des suspensions de cellules simples de celui-ci de pas plus de 10 semaines de développement, en excluant des cellules pancréatiques embryonnaires humaines,
(b) introduire une quantité efficace de tissu pancréatique humain ou de suspensions de cellules simples de celui-ci obtenu(es) dans l'étape (a) dans la capsule rénale d'un animal non humain diabétique non obèse/à déficit immunitaire combiné sévère (DNO/*dics*),
(c) laisser les cellules pancréatiques humaines se développer, se différencier et régénérer au moins une fonction pancréatique ; et
(d) recueillir les cellules pancréatiques humaines obtenues à l'étape (b) à différentes périodes du temps, et
(e) éventuellement cultiver *in vitro* les cellules obtenues à l'étape (c).

2. Utilisation des cellules pancréatiques obtenues par le procédé selon la revendication 1 pour étudier le développement du pancréas.

3. Utilisation des cellules pancréatiques obtenues par le procédé selon la revendication 1 pour étudier le développement physiopathologique du diabète.


### SEQUENCE LISTING

<110> UNIVERSITE PARIS 7
   CENTRE NATIONAL DE LA RECHERCHE SCIENTIFIQUE (CNRS)
<120> METHOD OF PRODUCING HUMAN BETA CELL LINES
<130> 343 973
<140>
   <141>
<160> 2
<170> PatentIn Ver. 2.1
<210> 1
   <211> 28
   <212> DNA
   <213> Artificial Sequence
<220>
   <223> Description of Artificial Sequence: Primer MluI RIP
<400> 1
   cgacgcgtgg acacagctat cagtggga 28
<210> 2
   <211> 25
   <212> DNA
   <213> Artificial Sequence
<220>
   <223> Description of Artificial Sequence: Antisense primer BamHI RIP
<400> 2
   cgggatccta gggctggggg ttact 25